# EUROPÄISCHE PATENTSCHRIFT

(11) **EP 1 543 315 B1**
(45) Veröffentlichungstag und Bekanntmachung des Hinweises auf die Patenterteilung: **14.02.2007**
(21) Anmeldenummer: 03753466.6
(22) Anmeldetag: 26.09.2003
(51) Int. Cl.: G01N 21/77, G01N 21/55

(54) **VORRICHTUNG ZUR DETEKTION MINDESTENS EINES IN EINER ZU UNTERSUCHENDEN PROBE ENTHALTENEN LIGANDEN**
DEVICE FOR THE DETECTION OF AT LEAST ONE LIGAND CONTAINED IN A SAMPLE THAT IS TO BE ANALYZED
DISPOSITIF PERMETTANT DE DETECTER UN LIGAND CONTENU DANS UN ECHANTILLON A ANALYSER

(30) Priorität: 27.09.2002 DE 10245435
(43) Veröffentlichungstag der Anmeldung: 22.06.2005
(73) Patentinhaber: Micronas GmbH, 79108 Freiburg i. Br. (DE)
(72) Erfinder: LEHMANN, Mirko, 79117 Freiburg (DE); MÜLLER, Claas, 79104 Freiburg (DE); KLAPPROTH, Holger, 79108 Freiburg (DE); FREUND, Ingo, 79235 Vogtsburg-Oberrotweil (DE)
(74) Vertreter: Huwer, Andreas
(86) Internationale Anmeldenummer: PCT/EP2003/010736
(87) Internationale Veröffentlichungsnummer: WO 2004/031750

(56) Entgegenhaltungen:
- WO-A-01/84197
- WO-A-95/33197
- WO-A-95/33198
- WO-A-97/06422
- DE-A- 19 947 616
- US-A- 5 936 730

## Beschreibung

Die Erfindung betrifft eine Vorrichtung nach dem Oberbegriff von Anspruch 1.

Eine derartige eine Vorrichtung ist aus DE 199 47 616 A1 bekannt. Sie weist zur Detektion eines in einer zu untersuchenden Probe enthaltenen Uganden einen optischen Wellenleiter auf, an dessen Oberfläche mehrere Detektionsfelder angeordnet sind. In den Detektionsfeldem sind unterschiedliche Rezeptoren, die beim Kontaktieren des Liganden eine spezifische Bindung mit dem Liganden eingehen, direkt oder indirekt immobilisiert. Die Vorrichtung weist zur evaneszenten Anregung der Emission von Lumineszenzstrahlung In Abhängigkeit von der Bindung des Liganden an den Rezeptor eine optische Strahlungsquelle zur Einkoppelung von Anregungsstrahlung in den Wellenleiter auf. Die Vorrichtung hat außerdem einen Halbleiterchip, der für die einzelnen Detektionsfelder jeweils wenigstens einen auf einem Halbleitersubstrat angeordneten Strahlungsempfänger zur ortsaufigelösten Detektion der Lumineszenzstrahlung aufweist.

Bei einer aus WO 95/33197 A1 bekannten Vorrichtung ist an der den Detektionsfeldem abgewandten Rückseite des Wellenleiters als Strahlungsempfänger eine CCD-Kamera vorgesehen. Der Strahlungsempfänger ist von dem Wellenleiter beabstandet und zwischen dem Wellenleiter und dem Strahlungsempfänger sind mehrere Linsen und ein Interferenzfilter angeordnet. Zur Messung der Absorption in dem Wellenleiter Ist zusätzlich ein weiterer Strahlungsempfänger vorgesehen. Dieser ist ebenfalls von dem Wellenleiter beabstandet

Bei einer aus WO 95/33198 A1 bekannten Vorrichtung wird optische Strahlung über ein Einkoppelgitter in einen Wellenleiter eingekoppelt und über ein von dem Einköppelgitter beabstandetes Auskoppelgitter aus dem Wellenleiter ausgekoppelt. Eine Strahlungsquelle und ein Strahlungsempfänger sind in einem vergleichsweise großen Abstand zu dem Wellenleiter und einem darunter befindlichen Substrat angeordnet. Zwischen dem Einkoppelgitter und dem Auskoppelgitter Ist das licht parallel zur Ebene des Wellenleiters geführt. Die Vorrichtung ermöglicht daher keine ortsaufgelöste Messung.

Aus DE 100 02 566 A1 Ist ferner einer Vorrichtung bekannt, die an der Oberfläche eines planaren Licht-Wellenleiters mehrere Messstellen aufweist, an denen als Rezeptoren spezifische Nukleinsäuren immobilisiert sind Mit den Rezeptoren wird eine zu untersuchende flüssige Probe in Kontakt gebracht, die zu den Rezeptoren komplementäre Nukleinsäuren enthält. Diese binden jeweils an die dazu passenden spezifischen Rezeptoren. Die so gebildeten, die zueinander komplementären Nukleinsäuren aufweisenden Rezeptor-Liganden-Komplexe werden mit einem Lumineszenzstoff markiert. Mit Hilfe zB. einer Laserdiode wird eine Anregungsstrahlung erzeugt und in den Licht-Wellenleiter eingekoppelt. Durch Totalreflexion an der Grenzfläche des Wellenleiters wird in einer an die Grenzfläche angrenzenden Schicht der Probe ein elektromagnetisches Feld erzeugt, das sogenannte Evaneszenzfeld. Dieses dringt nur einige hundert Nanometer von der Grenzfläche in die flüssige Probe ein. Durch das Evaneszenzfeld werden nahezu ausschließlich die an der Oberfläche des Wellenleiters gebundenen Lumineszenzstoffe zur Abgabe von Lumineszenzstrahlung angeregt. Zum Nachweis der in der Probe enthaltenen Nukleinsäuren wird die Lumineszenzstrahlung mit Hilfe einer CCD-Kamera ortsaufgelöst detektiert. Die CCD-Kamera ist an der den Rezeptoren abgewandten Rückseite des Wellenleiters angeordnet. Sie weist eine Abbildungsoptik auf, welche die einzelnen, an der Oberfläche des Wellenleiters befindlichen Messstellen jeweils auf ein Detektorelement eines CCD-Sensors abbildet. Die Vorrichtung hat den Nachteil, dass sie noch relativ viele Systemkomponenten aufweist und daher entsprechend teuer ist. Ungünstig ist außerdem, dass die Vorrichtung vergleichsweise große Abmessungen aufweist. Schließlich ist auch die Messempflndlichkeit der Vorrichtung noch verbesserungsfähig.

Aus WO 97/06422 A1 ist eine gattungsfremde Vorrichtung zur Detektion einer chemischen oder biochemischen Substanz bekannt, die einen optischen Wellenleiter, eine optische Strahlungsquelle zur Einkopplung von Anregungsstrahlung in den Wellenleiter und einen Halbleiterchip hat, der einen auf einem Halbleitersubstrat angeordneten, Strahlungsempfänger aufweist, der von dem Wellenleiter beabstandet ist. Der Wellenleiter ist monolithisch mit dem Halbleitersubstrat integriert In das Volumen des Wellenleiters sind lichtabsorblerende Reagenzien dispergiert Das von der Strahlungsquelle ausgesandte Ucht ist zwischen der Strahlungsquelle und dem Strahlungsempfänger In dem Wellenleiter etwa parallel zur Ebene des Halbleitersubstrats geführt. Es wird also die Absorption entlang einer von der Strahlungsquelle zu dem Strahlungsempfänger In dem Wellenleiter verlaufenden Messstrecke gemessen. Die Vorrichtung ermöglicht daher keine ortsaufgelöste Messung.

Es besteht deshalb die Aufgabe, eine Vorrichtung der eingangs genannten Art zu schaffen, die bei einem einfachen und kostengünstigen Aufbau eine kompakte Baugröße ermöglicht

Diese Aufgabe wird mit den Merkmalen des Patentanspruchs 1 gelöst

Die Strahlungsempfänger sind direkt an der dem Rezeptor abgewandten Rückseite des Wellenleiters und somit in entsprechend geringem Abstand zu dem wenigstens einen Rezeptor angeordnet. Somit ergibt sich eine sehr kompakte und flache Vorrichtung, die beispielsweise die Form eines Plättchens aufweisen kann. Aufgrund des geringen Abstands zwischen dem Rezeptor und dem Strahlungsempfänger kann eine aufwändige und teuere Abblidungsopfik zwischen dem Rezeptor und dem Strahlungsempfänger eingespart werden. Die von einem an dem Rezeptor angelagerten Lumineszenzstoff emittierte Lumineszenzstrahlung kann in einem großen Raumwinkelsegment detektiert werden. Es ergibt sich also eine einfach aufgebaute, kostengünstig herstellbare Vorrichtung mit großer Messempfindlichkelt

Die von einem an den Rezeptor gebundenen Uganden oder einem an diesem angeordneten Lumineszenzstoff ausgesandte Lumineszenzstrahlung verläuft etwa orthogonal zur Erstreckungsrichtung des Wellenleiters, wodurch eine gute Übertragung der Lumineszenzstrahlung durch den Wellenleiter hindurch In den Strahlungsempfänger erreicht wird. Die Lumineszenzstrahlung wird also ohne Umwege direkt zu dem Strahlungsempfänger geleitet, wodurch eine hohe Detektionsempfindlichkeit der Vorrichtung ermöglicht wird. Die Vorrichtung ermöglicht eine ortsaufgelöste Detektion von an der Oberfläche des Wellenleiters befindlichen Rezeptor-Liganden-Komplexen. Dabei kann die Probe gleichzeitig auf das Vorhandensein mehrerer unterschiedlicher Liganden untersucht werden Es ist aber auch denkbar, dass mindestens eine Gruppe von Detektionsfeldem die gleichen Rezeptoren aufweist. Die Messwerte der einzelnen Strahlungsempfänger der Gruppe können dann gemittelt, gefiltert und/oder zu Kontrollzwecken miteinander verglichen werden. Wenn mit Hilfe der Vorrichtung die Konzentration des Uganden in der Probe bestimmt werden soll, Ist es vorteilhaft, wenn die Rezeptoren von wenigstens zwei Detektionsfeldem eine unterschiedliche Affinität zu dem Liganden aufweisen. Die Konzentration des Liganden kann dann in einem breiten Konzentrationsbereich gemessen werden, ohne dass die Probe für die Messung verdünnt oder aufkonzentriert werden muss. Der Wellenleiter kann sich unterbrechungsfrei über die Strahlungsempfänger erstrecken. Dadurch kann bei der Herstellung des Wellenleiters ein Maskierungsschritt eingespart werden. Die Strahlungsempfänger sind vorzugsweise zeilen- oder matrixförmig nebeneinander in das Halbleitersubstrat integriert. Unter Lumineszenz werden alle Emissionen von Strahlungsquanten verstanden, vor allem Leuchterscheinungen, wie Fluoreszenz oder Phosphoreszenz, die Stoffe nach quantenhafter Anregung zeigen.

Vorteilhaft ist, wenn die Topographie des Halbleiterchips zur Vermeidung einer unerwünschten Lichtauskopplung aus dem Wellenleiter derart ausgebildet ist dass die dem wenigstens einen Rezeptor gegenüberliegende Grenzfläche zwischen dem Halbleiterchip und dem Wellenleiter zwischen zwei parallel zur Erstreckungsebene des Halbleiterchips angeordneten Ebenen verläuft, deren Abstand kleiner ist als die Wellenlänge der Anregungsstrahlung, insbesondere kleiner als die Hälfte, vorzugsweise ein Viertel und gegebenenfalls ein Achtel der Wellenlänge der Anregungsstrohlung. Im Bereich des Wellenleiters Ist also die Topographie des Halbleiterchips im Wesentlichen eben ausgebildet ist, was eine verlustarme Führung der Anregungsstrahlung in dem Wellenleiter ermöglicht. In entsprechender Weise kann auch die den Rezeptoren zugewandte Grenzfläche des Wellenleiters zwischen zwei parallel zur Erstreckungsebene des Halbleiterchips angeordneten Ebenen verlaufen, deren Abstand kleiner ist als die Wellenlänge der Anregungsstrahlung, insbesondere kleiner als die Hälfte, vorzugsweise ein Viertel und gegebenenfalls ein Achtel der Wellenlänge der Anregungsstrahlung. An der Grenzfläche zu dem Wellenleiter kann der Halbleiterchip eine Oxidschicht aufweisen.

Bei einer anderen Ausführungsform der Erfindung ist der Wellenleiter über wenigstens eine Bondstelle mit dem Halbleiterchip verbunden: Auch bei dieser Ausführungsform kann der Wellenleiter ein dünnes Kunststoffplättchen sein, das vorzugsweise eine Dicke von weniger als einem Millimeter aufweist.

Bei einer zweckmäßigen Ausgestaltung der Erfindung Ist der Wellenleiter als Dünnfilmschicht ausgebildet, die vorzugsweise aus einem transparenten Polymerwerkstoff besteht, insbesondere aus Polystyrol. Dabei ist der wenigstens eine Rezeptor vorzugsweise unmittelbar auf der Dünnfilmschicht angeordnet. Der Wellenleiter kann aber auch aus einem anderen Werkstoff bestehen, beispielsweise aus Spin-On-Glas. Mit Hilfe der Dünnfilmtechnik lässt sich der Wellenleiter mit einer Dicke von weniger als 100 Mikrometern herstellen. Auf den Strahlungsempfänger trifft dann ein entsprechend großer Anteil der von dem an den Rezeptor gebundenen Lumineszenzstoff abgegebenen Lumineszenzstrahlung auf Für den Nachweis des Liganden In der Probe wird somit nur eine geringe Probenmenge benötigt Bei der Herstellung der Vorrichtung kann der Wellenleiter durch Tauchbeschichten oder im Schleuderverfahren auf einfache Weise auf den Halbleiterchip aufgebracht werden.

Bei einer besonders vorteilhaften Ausführungsform der Erfindung Ist der Wellenleiter durch eine Metalloxidschicht gebildet, insbesondere eine Sillzlumdioxidschicht (SIO₂) oder eine Tantalpentoxidschicht (Ta₂O₅.) Der Wellenleiter lässt sich dann mit Hilfe von Standardprozessen der Halbleiterfertigung, wie z.B. durch Plasmaoxidation oder Chemical Vapour Deposition (CVD), kostengünstig herstellen. Dabei kann die Dicke des Wellenleiters weniger als 10 Mikrometer betragen, so dass nahezu die Hälfte - der von einem an der Oberfläche des Wellenleiters gebundenen Lumineszenzstoff emittierten Lumineszenzstrahlung auf den Strahlungsempfänger auftrifft. Es ergibt sich also eine entsprechend hohe Messempfindlichkeit, weshalb für die Untersuchung der Probe bereits kleinste Mengen Probenmaterial ausreichen. Die Oxidschicht kann eine Oberflächenstruktur haben, an welcher der wenigstens eine Rezeptor unmittelbar anhafteten kann. Dadurch kann eine Haftvermittlerschicht zwischen dem Wellenleiter und dem wenigstens einen Rezeptor eingespart werden. Es ist aber auch möglich, dass der Wellenleiter als Sillzium-Nitrid-Schicht (Si₃N₄) ausgebildet ist.

Besonders vorteilhaft ist, wenn die optische Strahlungsquelle als Halbleiterstrahlungsquelle ausgebildet und in den in den Halbleiterchip integriert ist. Die Vorrichtung ermöglicht dann einen noch kompakteren und kostengünstigeren Aufbau. Die Halbleiterstrahlungsquelle kann eine Laserdiode oder eine Leuchtdiode sein, welche die Anregungsstrahlung vorzugsweise in einem schmalbandigen Wellenlängenbereich emittiert, in dem der wenigstens eine Strahlungsempfänger unempfindlich ist.

Bei einer bevorzugten Ausgestaltung der Erfindung ist zum Einkoppeln der Anregungsstrahlung in den Wellenleiter im Abstrahlbereich der optischen Strahlungsquelle eine Einkoppelopfik vorgesehen, die vorzugsweise einstückig mit dem Wellenleiter ausgebildet ist und insbesondere wenigstens ein Prisma, ein optisches Gitter und/oder einen Umlenksplegel aufweist Die Strahlungsquelle kann an der den Rezeptoren abgewandten Rückseite des Wellenleiters mit ihrer Abstrahlseite der Einkoppeloptik des Wellenleiters zugewandt angeordnet sein. An der Einkoppeloptik wird dabei die von der Strahlungsquelle ausgesandte Anregungsstrahlung derart abgelenkt dass sie unter einem Winkel in den Wellenleiter eintritt, der so gewählt ist, dass die Strahlung unter Ausnutzung der Totalreflexion In dem Wellenleiter geführt wird. Somit ergibt sich eine verlustarme Führung der Anregungsstrahlung von der Strahlungsquelle zu dem wenigstens einen Rezeptor.

Zweckmäßigerweise sind die Detektionsfelder derart voneinander beabstandet und relativ zu den Strahlungsempfängern positioniert, dass die einzelnen Strahlungsempfänger im Wesentlichen keine Lumineszenzstrahlung von einem Detektionsfeld eines anderen Strahlungsempfängers empfangen. Somit wird eine hohe Übersprechdämpfung zwischen den aus den einzelnen Detekfionsfeldern und den diesen jeweils zugeordneten Strahlungsempfängern bestehenden Messanordnungen erreicht.
Bei einer vorteilhaften Ausführungsform der Erfindung ist der wenigstens eine Rezeptor in der Innenhöhlung einer Durchflussmesskammer angeordnet, die zumindest eine Einlass- und eine Auslassöffnung aufweist, wobei das Halbleitersubstrat vorzugsweise einen Wandungsbereich der Durchflussmesskammer bildet. In der Durchflussmesskammer können dann Biomoleküle oder Biokomponenten untersucht und über die Ein- und Auslassöffnung mit einer Nährflüssigkeit versorgt werden. Das Biomolekül kann Nukleinsäuren oder Derivate davon (DNA, RNA, PNA, LNA, Oligonukleotide, Plasmide, Chromosomen), Peptide, Proteine (Enzym, Protein, Oligopeptide, zelluläre Rezeptorproteine und deren Komplexe, Peptidhormone, Antikörper und deren Fragmente), Kohlenhydrate und deren Derivate, insbesondere glykosylierte Proteine und Glycoside, Fette, Fettsäuren und/oder Lipide umfassen.

Zum Temperieren der Durchflussmesskammer kann eine Heiz- und/oder Kühlvorrichtung vorgesehen sein, die vorzugsweise ein Peltier-Element aufweist. Die Heizvorrichtung ist vorzugsweise als Dünnfilmheizung ausgebildet Die Vorrichtung kann dann mehrfach verwendet werden, indem nach Durchführung einer Messung die Temperatur in der Durchflussmesskammer soweit erhöht wird, dass sich sämtliche an die Rezeptoren gebundenen Liganden von den Rezeptoren ablösen. Die Liganden können dann durch Zuführen eines Spülmediums über die Auslassöffnung aus der Durchflussmesskammer herausgespült werden. Dabei wird der Spülvorgang solange aufrechterhalten, bis die Strahlungsempfänger keine Lumineszenzstrahlung mehr detektieren. Danach kann über die Einlassöffnung eine neue Probe in die Durchflussmesskammer eingebracht und untersucht werden. Die Vorrichtung kann aber auch zur Bestimmung des Schmelzpunktes und/oder der Bindungskonstanten eines Liganden, wie z.B. einer DNA-Sequenz, verwendet werden. Dabei wird die Anbindung des Liganden an die Rezeptoren als Funktion der Temperatur und/oder der Zeit gemessen. Aus der so erhaltenen Schmelzkurve kann der Schmelzpunkt der DNA-Sequenz, also die Temperatur, bei der die Hälfte einer ursprünglich doppelsträngigen DNA-Sequenz einzelsträngig vorliegt, ermittelt werden. Anhand des Schmelzpunktes können Mutationen in DNA-Sequenzen, die beispielsweise bei Erbkrankheiten auftreten können, erkannt werden.

Bei einer zweckmäßigen Ausgestaltung der Erfindung ist in der Durchflussmesskammer wenigstens ein Reagenz und/oder Reaktionspartner zum Nachweisen der Bindung des wenigstens einen Liganden an den mindestens einen Rezeptor deponiert. Die Durchflussmesskammer ist dann fertig für die Verwendung, d.h. für die Messung braucht nur noch die zu untersuchende Probe über die Einlassöffnung in die Messkammer eingefüllt zu werden. Das Reagenz und/oder der Reaktionspartner ist vorzugsweise lyophilisiert und kann beispielsweise auf die Innenwand der Messkammer aufgedruckt sein. Vorzugsweise ist dabei ein stabilisierendes Agens, wie. z.B. Trehalose, Poly (2-hydroxyethl) methacrylat (pHEMA) oder bovines Serum Albumin (BSA), vorgesehen.

Nachfolgend sind Ausführungsbeispiele der Erfindung anhand der Zeichnung näher erläutert. Es zeigen zum Teil stärker schematisiert
- Fig. 1: einen Querschnitt durch eine Vorrichtung zur Detektion von in einer zu untersuchenden Probe enthaltenen Liganden, mit einer Durchflussmesskammer,
- Fig. 2 und 6: einen Querschnitt durch einen einen optischen Wellenleiter aufweisenden Wandungsbereich der Durchflussmesskammer, wobei auf dem Wellenleiter Rezeptoren immobilisiert sind, an die Liganden gebunden sind, die Indirekt mit einem Lumineszenzstoff markiert sind,
- Fig. 3: eine Darstellung ähnlich Fig. 2, wobei der Lumineszenzstoff mit Hilfe von Anregungs-Strahlung zur Abgabe von Lumineszenz-Strahlung angeregt wird und wobei die Anregungs- und die Lumineszenz-Strahlung schematisch in Form von Strahlen dargestellt sind,
- Fig. 4: eine Aufsicht auf einen Teilbereich eines Halbleiterchips, und
- Fig. 5: einen Querschnitt durch einen Teilbereich des Halbleiterchips und den daraufangeordneten Wellenleiter.

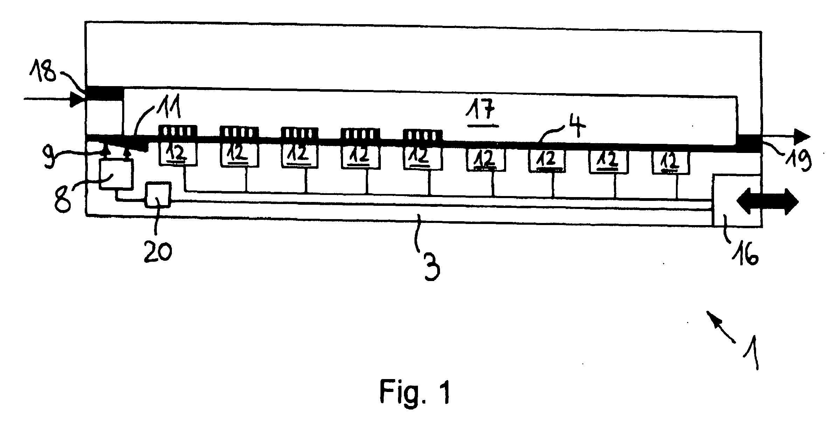

Eine im Ganzen mit 1 bezeichnete Vorrichtung zur Detektion mindestens eines in einer zu untersuchenden, im wesentlichen flüssigen Probe enthaltenen Liganden 2 weist einen Halbleiterchip 3 auf, der mit Methoden der Halbleitertechnik mit einem optischen Wellenleiter 4 integriert ist (Fig. 1). Der Wellenleiter kann beispielsweise aus einem Polymerwerkstoff bestehen.

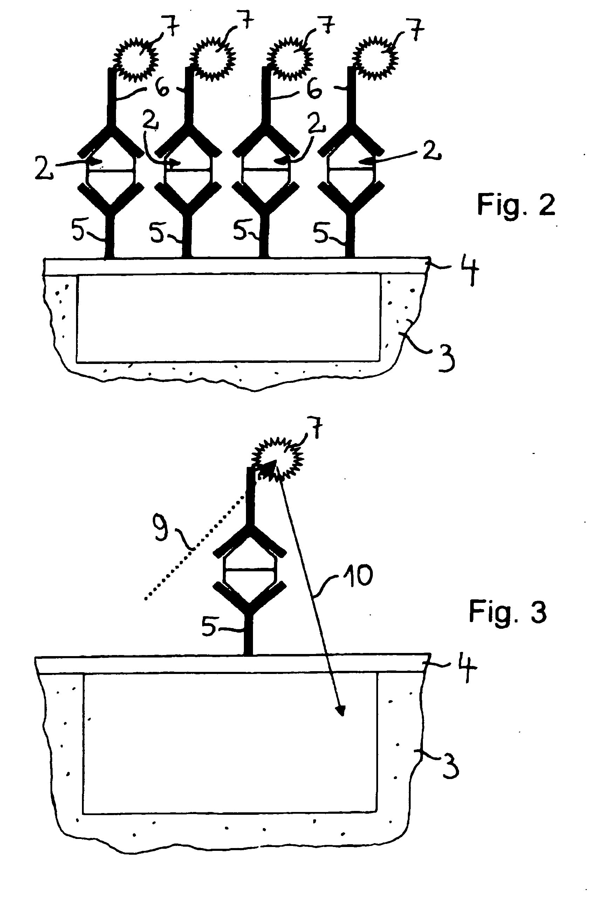

In Fg. 2 ist erkennbar, dass an der Oberfläche des Wellenleiters 4 Rezeptoren 5 immobilisiert, die beim Kontaktieren des Uganden 2 an diesen binden. Die immobilisierung der Rezeptoren 5 kann beispielsweise durch eine Silanisierung oder eine auf dem Wellenleiter 4 angeordnete Polyimidschicht erreicht werden, an welcher die Rezeptoren 5 anhaften. Die Rezeptoren 5 können auf den Wellenleiter 4 bzw. die darauf befindliche Polyimidschicht aufgedruckt sein. Bei dem Ausführungsbeispiel nach Fig. 2 sind die Rezeptoren 5 Antikörper gegen ein bestimmtes Epitop des Liganden 2. Nach Bindung des Epitops an den Rezeptor 5 wird der so gebildete, aus dem Epitop und dem Rezeptor 5 bestehende Antikörperkomplex mittels eines zweiten, an das erste Epitop bindenden Antikörpers 6 markiert. Dieser Antikörper 6 ist direkt oder indirekt mit einem Lumineszenzstoff 7 markiert.

In Fig. 1 ist erkennbar, dass in den Halbleiterchip 3 eine optische HalbleiterStrahlungsquelle 8, wie zum Beispiel eine Laser- oder Leuchtdiode, integriert ist. Das Spektrum der von der Strahlungsquelle 8 emittierten Strahlung 9 weist mindestens eine Anregungswellenlänge auf, bei welcher der Lumineszenzstoff 7 zur Abgabe von Lumineszenzstrahlung 10 angeregt wird. Zum Einkoppeln der Anregungsstrahlung 9 in den Wellenleiter 4 ist im Abstrahlbereich der Strahlungsquelle 8 eine Einkoppeloptik 11 vorgesehen ist, die in der Zeichnung nicht näher dargestellte Mikroprismen aufweist, welche die von der Strahlungsquelle 8 ausgesandte Anregungsstrahlung derart umlenken, dass sie unter Ausnutzung der Totalreflexion in dem Wellenleiter 4 geführt ist. Durch die Totalreflexion an der Grenzfläche des Wellenleiters 4 wird in dem optisch dünneren Medium, nämlich der Probe, ein elektromagnetisches Evaneszenzfeld erzeugt, wodurch die an der Oberfläche des Wellenleiters 4 gebundenen Lumineszenzstoffe 7 zur Abgabe der Lumineszenzstrahlung 10 angeregt werden. Da das Evaneszenzfeld nur einige hundert Nanometer tief in die Probe eindringt, werden nahezu ausschließlich die an der Oberfläche des Wellenleiters gebundenen Lumineszenzstoffe 7 zur Abgabe der Lumineszenzstrahlung 10 angeregt, während in der Probe enthaltene ungebundene Lumineszenzstoffe 7 praktisch nicht zu der Lumineszenzstrahlung beitragen.

Zur Detektion der Lumineszenzstrahlung 10 sind mehrere, jeweils als Halbleiterbauelemente ausgebildete optische Strahlungsempfänger 12 in den Halbleiterchip 3 integriert. Die Strahlungsempfänger 12 sind an der den Rezeptoren 5 abgewandten Rückseite des für die Lumineszenzstrahlung 10 durchlässigen Wellenleiters 4 angeordnet. Die Lumineszenzstrahlung10 trifft also ohne Zwischenschaltung einer Abbildungsoptik direkt auf die Strahlungsempfänger 12 auf Die Vorrichtung weist dadurch einen kompakten und kostengünstigen Aufbau auf.

Bei dem Lumineszenzstoff 7 handelt es sich um einen aufwärtskonvertierenden Lumineszenzstoff. Derartige Lumineszenzstoffe sind aus EP 0 723 146 A1 bekannt. Als Beispiele für aufwärtskonverfierende Lumineszenzstoffe seinen die Farbstoff BND der Dyomics GmbH, Jena und IR-140 erwähnt. Anders als abwärtskonvertierende Lumineszenzstoffe beziehen aufwärtskonvertierende Lumineszenzstoffe die für die Quantenemission benötigte Energie nicht aus einem einzigen sondern aus mehreren Quanteneffekten. Abwärtskonvertierende Lumineszenzstoffe weisen daher im Vergleich zu abwärtskonverterenden Lumineszenzstoffen eine wesentlich größere Stokes-Verschiebung auf, bei welcher die Wellenlänge der Anregungsstrahlung beispielsweise etwa doppelt so groß sein kann wie die Wellenlänge der Lumineszenz-Strahlung. Dadurch ist es möglich, als Strahlungsquelle eine Infrarot-HalbleiterStrahlungsquelle 8 vorzusehen, die bei kompakten Abmessungen eine hohe Strahlungsintensität ermöglicht. Das Infrarotlicht derartiger Halbleiter-Strahlungsquellen 8 hat außerdem den Vorteil, dass gegenüber kurzwelligerer optischer Strahlung weniger Streueffekte auftreten. Mit Hilfe des aufwärtskonvertierenden Lumineszenzstoffs 7 kann die von der Strahlungsquelle 8 abgegebene optische Strahlung in sichtbares Licht oder in nahes Infrarot-Licht konvertiert werden, für das die Strahlungsempfänger 12 eine hohe Detektionsempfindlichkeit aufweisen. Für die Anregungsstrahlung 9 sind die Strahlungsempfänger 12 unempfindlich.

In Fig. 2 und 3 ist erkennbar, dass sich der Wellenleiter 4 bis über die Strahlungsempfänger 12 erstreckt und dass die Rezeptoren 5 dem Strahlungsempfänger 12 direkt gegenüberliegend an der Oberfläche des Wellenleiters 4 angeordnet sind. Somit kann die Lumineszenzstrahlung 10 auf direktem Wege von dem Lumineszenzstoff 7 zu den Strahlungsempfängern 12 gelangen.

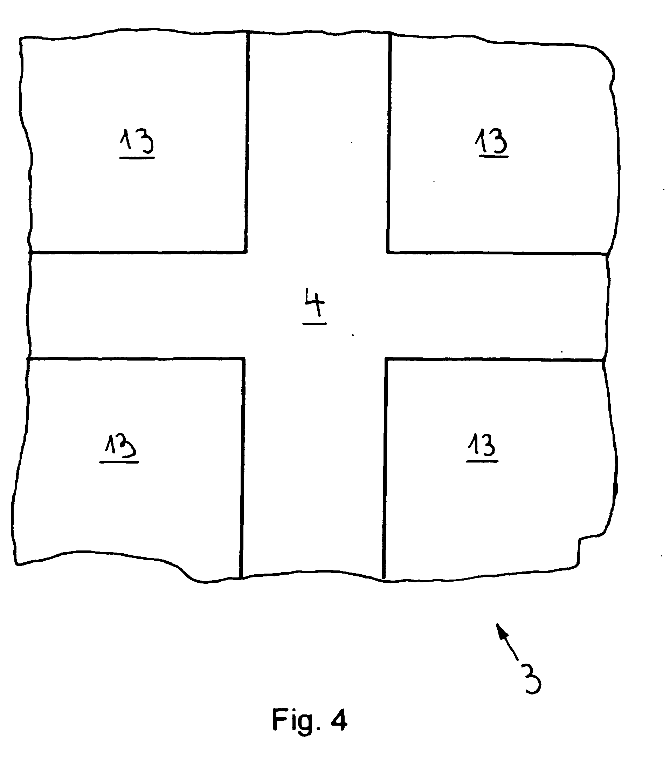

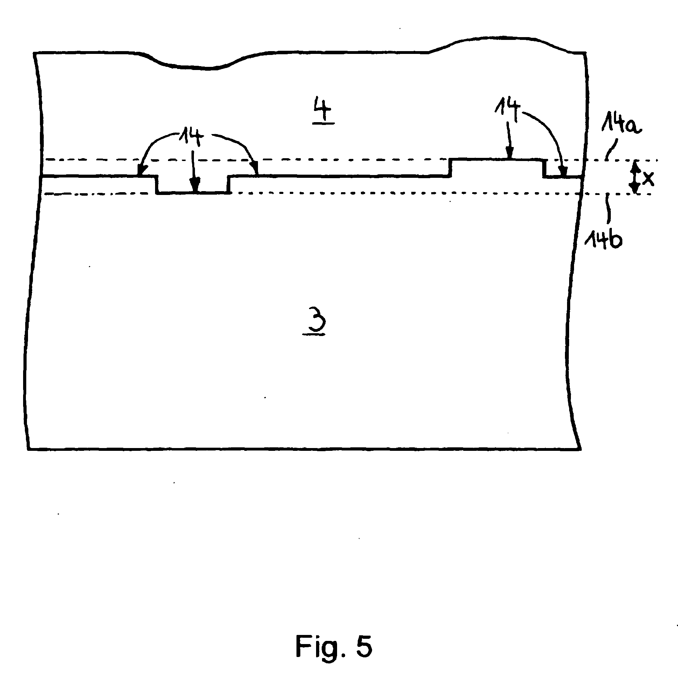

Bei dem in Fig. 4 und 5 gezeigten Ausführungsbeispiel grenzt der Wellenleiter 4 direkt an den Halbleiterchip 3 an. Der Wellenleiter 4 weist Unterbrechungen auf, in denen Strukturen 13 für eine elektronische Schaltung angeordnet sind. Diese weist Leiterbahnen auf, die mit den Strahlungsempfängern 12 verbunden sind. Die Topographie des Halbleiterchips in dem an den Wellenleiter 4 angrenzenden Halbleiterchip-Bereich derart ausgebildet ist, dass die den Rezeptoren 5 gegenüberliegende Grenzfläche 14 zwischen dem Halbleiterchip 3 und dem Wellenleiter 4 zwischen zwei gedachten, jeweils parallel zur Erstreckungsebene des Halbleiterchips 3 angeordneten Ebenen 14a, 14b verläuft, deren Abstand x kleiner ist als ein Achtel der Wellenlänge der Anregungsstrahlung 9. Dadurch wird eine unerwünschte Lichtauskopplung aus dem Wellenleiter 4 an der Grenzfläche 14 weitestgehend vermieden. Strukturen, die eine von einer Ebene abweichende Oberflächen-Topographie des Halbleiterchips 3 erfordern, wie zum Beispiel Leiterbahnen aus Aluminium, sind im Wesentlichen seitlich neben dem Wellenleiter 4 angeordnet. Bei dem Ausführungsbeispiel nach Fig. 5 ist der Wellenleiter 4 ist durch eine Halbmetalloxid-Schicht gebildet, die sich in einem oberflächennahen Bereich flächig auf einem Halbleitersubstrat des Halbleiterchips 3 erstreckt und etwa parallel zur Erstreckungsebene des Halbleitersubstrats verläuft. Das Halbleitersubstrat kann beispielsweise aus Silizium bestehen.

In Fig. 1 ist erkennbar, dass die Strahlungsempfänger 12 über Leiterbahnen mit einer in den Halbleiterchip 3 Integrierten Ansteuerungs- und Auswerteeinrichtung 17 verbunden sind. Die Auswerteeinrichtung 17 hat eine In der Zeichnung schematisch dargestellte Schnittstelleneinrichtung zum Verbinden mit einer übergeordneten Anzeige- und/oder Auswerteeinheit, beispielsweise einem Mikrocomputer.

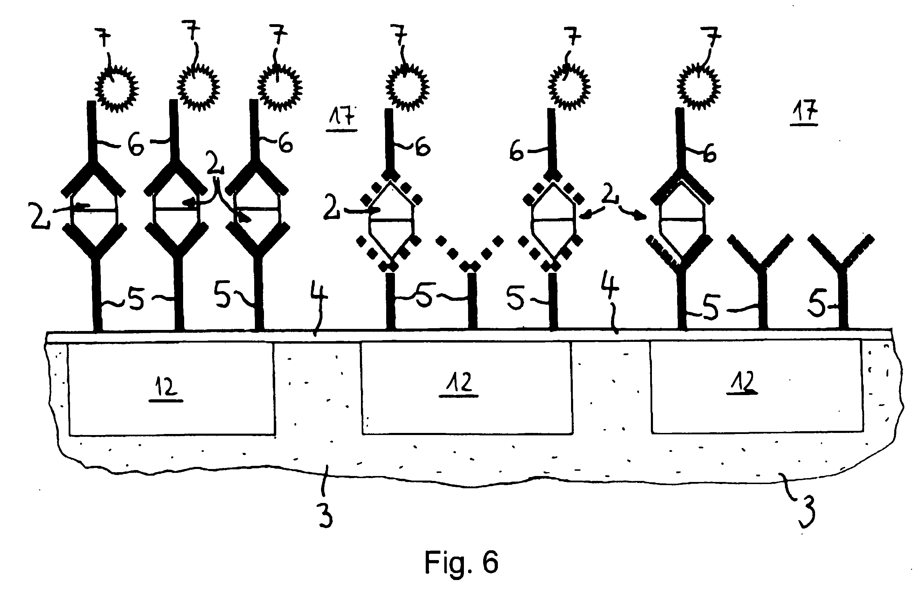

In Fig. 6 ist erkennbar, dass mehrere Strahlungsempfänger 12 matrixförmig nebeneinander In das Halbleitersubstrat integriert sind. Im Detektionsbereich der einzelnen Strahlungsempfänger 12 ist jeweils ein Detektionsfeld mit mehreren Rezeptoren 5 angeordnet. Die einzelnen Detekfionsfelder haben unterschiedliche Rezeptoren 5, die jeweils mit einem bestimmten Liganden 2 eine Bindung eingehen können.

In Fig. 2 bis 4 sind die Rezeptoren gegenüber den Strahlungsempfängern 12 vergrößert dargestellt. Die Abstände zwischen zueinander benachbarten Detektionsfeldern einerseits und die Abstände zwischen den an die Rezeptoren 5 gebundenen Lumineszenzstoffen 7 und den ihnen jeweils zugeordneten Strahlungsempfängern 12 andererseits sind so gewählt, dass die einzelnen Strahlungsempfänger 12 im Wesentlichen keine Lumineszenzstrahlung von einem Detektionsfeld eines benachbarten Strahlungsempfängers empfangen können.

In Fig. 1 ist erkennbar, dass der Halbleiterchip 3 einen Wandungsbereich einer Durchflussmesskammer bildet, in deren Innenhöhlung 17 die Rezeptoren 5 angeordnet sind. Die Durchflussmesskammer hat eine Einlassöffnung 19 und eine Auslassöffnung 19. Die Einlassöffnung 19 ist mit einer in der Zeichnung nicht näher dargestellten Zuleitung für die Probe und die Auslassöffnung 19 mit einer Ableitung verbunden.

Erwähnt werden soll noch, dass die Strahlungsquelle 8 zum Modulieren der Anregungsstrahlung 9 mit einer Modulationseinrichtung 20 verbunden ist, die in den Halbleiterchip 3 integriert ist. Mit Hilfe der Modulationseinrichtung 20 kann die Anregungsstrahlung 9 beispielsweise getaktet ein- und ausgeschaltet werden, um in den Messsignalen der Strahlungsempfänger 12 eventuell enthaltene, durch Streulicht oder unspezifische optische Anregung verursachte Signalanteile bei der Signalauswertung berücksichtigen zu können. Die Modulationseinrichtung 20 ist zu diesem Zweck über eine Verbindungsleitung mit der Auswerteeinrichtung 16 verbunden.

Die Vorrichtung 1 zur Detektion mindestens eines in einer zu untersuchenden Probe enthaltenen Liganden 2 weist also einen optischen Wellenleiter 4 auf, an dessen Oberfläche wenigstens ein für den Liganden 2 spezifischer Rezeptor 5 direkt oder indirekt immobilisiert ist. Der Ligand 2 bindet beim Kontaktieren des Rezeptors 5 an diesen. Die Vorrichtung 1 hat mindestens eine optische Strahlungsquelle 8 zur Einkoppelung von Anregungsstrahlung 9 in den Wellenleiter 4. Die Strahlung 9 dient zur Anregung der Emission von Lumineszenzstrahlung 10 in Abhängigkeit von der Bindung des Liganden 2 an den Rezeptor 5. Zur Detektion der Lumineszenzstrahlung 10 ist in das Halbleitersubstrat eines Halbleiterchips 3 mindestens ein Strahlungsempfänger 12 integriert. Der Wellenleiter 4 ist monolithisch mit dem Halbleitersubstrat integriert oder als Wellenleiterschicht auf dieses aufgebracht.

## Patentansprüche

1. Vorrichtung (1) zur Detektion mindestens eines in einer zu untersuchenden Probe enthaltenen Liganden (2), mit einem optischen Wellenleiter (4), an dessen Oberfläche mehrere Detektionsfelder angeordnet sind, in denen unterschledliche Rezeptoren direkt oder Indirekt immobilisiert sind, die beim Kontaktieren des Liganden (2) eine spezifische Bindung mit dem Liganden (2) eingehen, mit mindestens einer optischen Strahlungsquelle (8) zur Einkoppelung von Anregungsstrahlung (9) in den Wellenleiter (4), zur evaneszenten Anregung der Emission von Lumineszenzstrahlung (10) in Abhängigkeit von der Bindung des Uganden (2) an den Rezeptor (5), und mit einem Halbleiterchip (3), der für die einzelnen Defiektionsfelder jeweils wenigstens einen auf einem Halbleitersubstrat angeordneten Strahlungsempfänger (12) zur ortsaufgelösten Detektion der Lumineszenzstrahlung (10) aufweist, **dadurch gekennzeichnet, dass** der Wellenleiter (4) monolithisch mit dem Halbleitersubstrat integriert ist oder als Wellenleiterschicht auf dem Halbleiterchip (3) angeordnet ist, und dass die den Detektionsfeldem zugeordneten Strahlungsempfänger (12) den Detektionsfeldem gegenüberliegend direkt an der den Detektionsfeldern abgewandten Rückseite des Wellenleiters nebeneinander in das Halbleitersubstrat integriert sind.

2. Vorrichtung (1) nach Anspruch 1, **dadurch gekennzeichnet, dass** die Topographie des Halbleiterchips (3) zur Vermeidung einer unerwünschten Lichtauskopplung aus dem Wellenleiter (4) derart ausgebildet ist, dass die dem wenigstens einen Rezeptor (5) gegenüberliegende Grenzfläche (14) zwischen dem Halbleiterchip (3) und dem Wellenleiter (4) zwischen zwei parallel zur Erstreckungsebene des Halbleiterchips (3) angeordneten Ebenen (14a, 14b) verlauft deren Abstand (x) kleiner ist als die Wellenlänge der Anregungsstrahlung (9), insbesondere kleiner als die Hälfte, vorzugsweise ein Viertel und gegebenenfalls ein Achtel der Wellenlänge der Anregungsstrahlung (9).

3. Vorrichtung (1) nach Anspruch 1 oder 2, **dadurch gekennzeichnet, dass** der Halbleiterchip (3) seitlich neben dem Wellenleiter (4) Strukturen (13) für eine elektronische Schaltung aufweist

4. Vorrichtung (1) nach einem der Ansprüche 1 bis 3, **dadurch gekennzeichnet, dass** der Wellenleiter (4) über wenigstens eine Bondstelle mit dem Halbleiterchip (3) verbunden ist.

5. Vorrichtung (1) nach einem der Ansprüche 1 bis 4, **dadurch gekennzeichnet, dass** der Wellenleiter (4) als Dünnfilmschicht ausgebildet ist, die vorzugsweise aus einem transparenten Polymerwerkstoff besteht, insbesondere aus Polystyrol.

6. Vorrichtung (1) nach einem der Ansprüche 1 bis 5, **dadurch gekennzeichnet, dass** der Wellenleiter (4) durch eine Metalloxidschicht gebildet ist insbesondere eine Slliziumdioxidschicht oder eine Tantalpentoxidschicht

7. Vorrichtung (1) nach einem der Ansprüche 1 bis 6, **dadurch gekennzeichnet, dass** die optische Strahlungsquelle (8) als Halbleiterstrahlungsquelle ausgebildet und in den Halbleiterchip (3) integriert ist.

8. Vorrichtung (1) nach einem der Ansprüche 1 bis 7, **dadurch gekennzeichnet, dass** zum Einkoppeln der Anregungsstrahlung (9) In den Wellenleiter (4) im Abstrahlbereich der optischen Strahlungsquelle (8) eine Einkoppeloptik (11) vorgesehen ist, die vorzugsweise einstückig mit dem Wellenleiter (4) ausgebildet ist und insbesondere wenigstens ein Prisma, ein optisches Gitter und/oder einen Umlenkspiegel aufweist.

9. Vorrichtung (1) nach einem der Ansprüche 1 bis 8, **dadurch gekennzeichnet, dass** die Detektionsfelder derart voneinander beabstandet und relativ zu den Strahlungsempfängern (12) positioniert sind, dass die einzelnen Strahlungsempfänger (12) Im Wesentlichen keine Lumineszenzstrahlung von einem Detektionsfeld eines anderen Strahlungsempföngers (12) empfangen.

10. Vorrichtung (1) nach einem der Ansprüche 1 bis 9, **dadurch gekennzeichnet, dass** der wenigstens eine Rezeptor (5) in der Innenhöhlung (17) einer Durchflussmesskammer angeordnet ist, die zumindest eine Einlassöffnung (19) und eine Auslassöffnung (19) aufweist, und dass der Halbleiterchip (3) vorzugsweise einen Wandungsbereich der Durchflussmesskammer bildet.

11. Vorrichtung (1) nach einem der Ansprüche 1 bis 10, **dadurch gekennzeichnet, dass** zum Temperieren der Durchflussmesskammer eine Helz- und/oder Kühlvorrichtung vorgesehen ist die vorzugsweise ein Peltier-Element aufweist.

12. Vorrichtung (1) nach einem der Ansprüche 1 bis 11, **dadurch gekennzeichnet, dass** in der Durchflussmesskammer wenigstens ein Reagenz und/oder Reaktionspartner zum Nachweisen der Bindung des wenigstens einen Uganden (2) an den mindestens einen Rezeptor (5) deponiert ist.

## Claims

1. Device (1) for the detection of at least one ligand (2) contained in a sample that is to be analysed, comprising an optical waveguide (4), at the surface of which are arranged a plurality of detection zones in which different receptors are immobilized directly or indirectly, which, upon contacting of the ligand (2) enter into a specific binding with the ligand (2), comprising at least one optical radiation source (8) for coupling excitation radiation (9) into the waveguide (4), for the evanescent excitation of the emission of luminescence radiation (10) in a manner dependent on the binding of the ligand (2) to the receptor (5) and comprising a semiconductor chip (3) having, for the individual detection zones, in each case at least one radiation receiver (12) arranged on a semiconductor substrate for the spatially resolved detection of the luminescence radiation (10), **characterized in that** the waveguide (4) is monolithically integrated with the semiconductor substrate or is arranged as a waveguide layer on the semiconductor chip (3), and **in that** the radiation receivers (12) assigned to the detection zones are integrated into the semiconductor substrate alongside one another in a manner lying opposite the detection zones directly at the rear side of the waveguide remote from the detection zones.

2. Device (1) according to Claim 1, **characterized in that** the topography of the semiconductor chip (3), for avoiding an undesirable coupling-out of light from the waveguide (4), is embodied in such a way that the interface (14) between the semiconductor chip (3) and the waveguide (4), said interface lying opposite the at least one receptor (5) runs between two planes (14a, 14b) which are arranged parallel to the plane of extent of the semiconductor chip (3) and the distance (x) between which is less than the wavelength of the excitation radiation (9), in particular less than half, preferably a quarter, and if appropriate an eighth, of the wavelength of the excitation radiation (9).

3. Device (1) according to Claim 1 or 2, **characterized in that** the semiconductor chip (3) has structures (13) for an electronic circuit laterally alongside the waveguide (4).

4. Device (1) according to one of Claims 1 to 3, **characterized in that** the waveguide (4) is connected to the semiconductor chip (3) by means of at least one bonding location.

5. Device (1) according to one of Claims 1 to 4, **characterized in that** the waveguide (4) is embodied as a thin-film layer which preferably comprises a transparent polymer material, in particular polystyrene.

6. Device (1) according to one of Claims 1 to 5, **characterized in that** the waveguide (4) is formed by a metal oxide layer, in particular a silicon dioxide layer or a tantalum pentoxide layer.

7. Device (1) according to one of Claims 1 to 6, **characterized in that** the optical radiation source (8) is embodied as a semiconductor radiation source and is integrated into the semiconductor chip (3).

8. Device (1) according to one of Claims 1 to 7, **characterized in that** a coupling-in optic (11) is provided for coupling the excitation radiation (9) into the waveguide (4) in the emission region of the optical radiation source (8), which optic is preferably embodied integrally with the waveguide (4) and has, in particular, at least one prism, an optical grating and/or a deflection mirror.

9. Device (1) according to one of Claims 1 to 8, **characterized in that** the detection zones are spaced apart from one another and positioned relative to the radiation receivers (12) in such a way that the individual radiation receivers (12) receive essentially no luminescence radiation from a detection zone of another radiation receiver (12).

10. Device (1) according to one of Claims 1 to 9, **characterized in that** the at least one receptor (5) is arranged in the inner cavity (17) of a flow measuring chamber and at least one inlet opening (19) and an outlet opening (19), and **in that** the semiconductor chip (3) preferably forms a wall region of the flow measuring chamber.

11. Device (1) according to one of Claims 1 to 10, **characterized in that** a heating and/or cooling device, which preferably has a Peltier element, is provided for regulating the temperature of the flow measuring chamber.

12. Device (1) according to one of Claims 1 to 11, **characterized in that** at least one reagent and/or reactant for detecting the binding of the at least one ligand (2) to the at least one receptor (5) is deposited in the flow measuring chamber.

## Revendications

1. Dispositif (1) pour la détection d'au moins un ligand (2) contenu dans un échantillon à étudier, lequel dispositif présente :
un conducteur d'ondes optiques (4) à la surface duquel sont disposées plusieurs surfaces de détection dans lesquelles différents détecteurs qui, lorsqu'ils entrent en contact avec le ligand (2), établissent une liaison spécifique avec le ligand (2), sont immobilisés directement ou indirectement,
au moins une source (8) de rayonnement optique qui injecte un rayonnement d'excitation (9) dans le conducteur d'ondes (4) pour exciter de manière évanescente l'émission d'un rayonnement luminescent (10) en fonction de la liaison du ligand (2) sur le récepteur (5),
une puce semi-conductrice (3) qui présente pour chacune des surfaces de détection au moins un récepteur de rayonnement (12) disposé sur un substrat semi-conducteur pour détecter le rayonnement luminescent (10) avec une résolution spatiale,
**caractérisé en ce que**
le conducteur d'ondes (4) est intégré monolithiquement au substrat semi-conducteur ou est disposé sur la puce semi-conductrice (3) sous la forme d'une couche de conducteur d'ondes et
**en ce que** les récepteurs de rayonnement (12) associés aux surfaces de détection sont intégrés directement dans le substrat semi-conducteur sur le côté arrière du conducteur d'ondes non tourné vers les surfaces de détection, les uns à côtés des autres et en face des surfaces de détection.

2. Dispositif (1) selon la revendication 1, **caractérisé en ce que** pour éviter une sortie indésirable de lumière hors du conducteur d'ondes (4), la topographie de la puce semi-conductrice (3) est configurée de telle sorte que la surface frontière (14) opposée au récepteur (5) au moins prévu et située entre la puce semi-conductrice (3) et le conducteur d'ondes (4) s'étend entre deux plans (14a, 14b) disposés parallèlement au plan de l'extension de la puce semi-conductrice (3), la distance (x) entre ces plans étant inférieure à la longueur d'onde du rayonnement d'excitation (9) et en particulier inférieure à sa moitié et représentant de préférence le quart et éventuellement le huitième de la longueur d'onde du rayonnement d'excitation (9).

3. Dispositif (1) selon la revendication 1 ou 2, **caractérisé en ce que**, à côté du conducteur d'ondes (4), la puce semi-conductrice (3) présente des structures (13) d'un circuit électronique.

4. Dispositif (1) selon l'une quelconque des revendications 1 à 3, **caractérisé en ce que** le conducteur d'ondes (4) est relié à la puce semi-conductrice (3) par au moins un emplacement de liaison.

5. Dispositif (1) selon l'une quelconque des revendications 1 à 4, **caractérisé en ce que** le conducteur d'ondes (4) est réalisé sous la forme d'une couche en film mince qui est de préférence constituée d'un matériau polymère transparent et en particulier de polystyrène.

6. Dispositif (1) selon l'une quelconque des revendications 1 à 5, **caractérisé en ce que** le conducteur d'ondes (4) est formé d'une couche d'oxyde métallique et en particulier d'une couche de dioxyde de silicium ou d'une couche de pentoxyde de tantale.

7. Dispositif (1) selon l'une quelconque des revendications 1 à 6, **caractérisé en ce que** la source (8) de rayonnement optique est configurée comme source semi-conductrice de rayonnement et est intégrée dans la puce semi-conductrice (3).

8. Dispositif (1) selon l'une quelconque des revendications 1 à 7, **caractérisé en ce qu'**une optique d'injection (11) qui est de préférence configurée d'un seul tenant avec le conducteur d'ondes (4) et qui présente en particulier au moins un prisme, une grille optique et/ou un miroir de renvoi, est prévue dans la zone d'émission de la source (8) de rayonnement optique pour injecter le rayonnement d'excitation (9) dans le conducteur d'ondes (4).

9. Dispositif (1) selon l'une quelconque des revendications 1 à 8, **caractérisé en ce que** les surfaces de détection sont disposées à distance les unes des autres et des récepteurs (12) de rayonnement, de telle sorte que les différents récepteurs (12) de rayonnement ne reçoivent sensiblement pas de rayonnement de luminescence provenant de la surface de détection d'un autre récepteur (12) de rayonnement.

10. Dispositif (1) selon l'une quelconque des revendications 1 à 9, **caractérisé en ce que** ledit au moins un récepteur (5) est disposé dans le creux intérieur (17) d'une chambre de mesure d'écoulement qui présente au moins une ouverture d'admission (19) et une ouverture de sortie (19) et **en ce que** la puce semi-conductrice (3) forme de préférence une partie de la paroi de la chambre de mesure d'écoulement.

11. Dispositif (1) selon l'une quelconque des revendications 1 à 10, **caractérisé en ce qu'**un dispositif de chauffage et/ou de refroidissement qui présente de préférence un élément Peltier est prévu pour contrôler la température de la chambre de mesure d'écoulement.

12. Dispositif (1) selon l'une quelconque des revendications 1 à 11, **caractérisé en ce qu'**au moins un réactif et/ou un partenaire de réaction est déposé dans la chambre de mesure d'écoulement pour détecter la liaison du ou des ligands (2) avec le ou les récepteurs (5).
